# FASCICULE DE BREVET EUROPEEN

(11) **EP 4 361 127 B1**
(45) Date de publication et mention de la délivrance du brevet: **31.12.2025**
(21) Numéro de dépôt: 24162955.9
(22) Date de dépôt: 01.04.2021
(51) Int. Cl.: C07C 303/22, C07C 309/49, C09B 11/10, C07C 213/08, C07C 303/06, C07C 215/74, A61K 49/04, A61P 35/00

(54) **PROCÉDÉ DE SYNTHÈSE DE COMPOSÉS TRIARYLMÉTHANE SULFONÉS**
VERFAHREN ZUR HERSTELLUNG VON SULFONIERTEN TRIARYLMETHANVERBINDUNGEN
PROCESS FOR THE SYNTHESIS OF SULFONATED TRIARYLMETHANE COMPOUNDS

(30) Priorité: 10.04.2020 FR 2003643
(43) Date de publication de la demande: 01.05.2024
(62) Demande divisionnaire de: 21715910.2
(73) Titulaire: Provepharm Life Solutions, 13013 Marseille (FR)
(72) Inventeur: QUERU, Stéphane, 13013 Marseille (FR); SAYAH, Babak, 13013 Marseille (FR); DRILLAUD, Nicolas, 13013 Marseille (FR); LOPEZ, Nicolas, 13013 Marseille (FR)
(74) Mandataire: Bandpay & Greuter

(56) Documents cités:
- WO-A1-2017/118882
- RU-C1- 2 654 862
- GAJOS RAFAL ET AL: "Preliminary results for interval feeding the orthogonal pressurized planar electrochromatography system with sample solution for its preparative separation", JOURNAL OF CHROMATOGRAPHY A, ELSEVIER, AMSTERDAM, NL, vol. 1499, 2 April 2017 (2017-04-02), pages 183 - 189, XP085009914, ISSN: 0021-9673, DOI: 10.1016/J.CHROMA.2017.03.088

## Description

### Domaine technique

La présente invention concerne un nouveau procédé de synthèse de composés comprenant une structure triarylméthane substituée par des groupements sulfonés, notamment un nouveau procédé de synthèse de colorants de la famille des composés triarylméthane sulfonés, en particulier du bleu patenté.

### État de la technique antérieure

Le bleu patenté (sel de 4-[[4-(diéthylamino)phényl]-(4-diéthylazaniumylidènecyclohexa-2,5-dièn-1-ylidène)méthyl]-6-hydroxybenzène-1,3-disulfonate) est un colorant comportant une structure triarylméthane qui existe sous deux formes : le sel de sodium [CAS 20262-76-4] et le sel de calcium [CAS 3536-49-0]. Ce colorant a été mentionné pour la première fois en 1897 par Ernst Erdmann et Hugo Erdmann, Justus Liebigs Annalen der Chemie, Vol 294 (3), 376-392. Dans les publications le concernant, il existe parfois une confusion entre le bleu patenté et le bleu d'isosulfan (sel de sodium du 2-[[4-(diéthylamino)phényl]-(4-diéthylazaniumylidènecyclohexa-2,5-dièn-1-ylidène)méthyl]benzène-1,4-disulfonate, CAS 68238-36-8 et 748080-29-7), bien que ces molécules soient de structures distinctes.

Le bleu patenté est disponible commercialement, mais les produits que l'on peut trouver sur le marché se caractérisent par une teneur en impuretés élevée. Notamment, on trouve des quantités importantes de composé monodeséthylé qui sont difficiles à séparer du bleu patenté par des méthodes classiques de purification. Ils se caractérisent également par une quantité significative de NaCl résiduel.

Le bleu patenté et le bleu d'isosulfan sont des colorants des tissus biologiques utilisés notamment comme agents de contraste pour la délimitation des vaisseaux lymphatiques et sont particulièrement utiles comme agents de diagnostic du cancer. Ils sont couramment utilisés dans une procédure de diagnostic appelée «cartographie des ganglions lymphatiques sentinelles». Cette méthode constitue un complément à la lymphographie pour la visualisation du système lymphatique drainant la région d'injection. Une application importante consiste à localiser les ganglions lymphatiques sentinelles chez les patientes atteintes d'un cancer du sein. L'ablation chirurgicale des tissus cancéreux, guidée par le bleu patenté ou par le bleu isosulfan, est également pratiquée.

Peu d'auteurs se sont intéressés à la synthèse du bleu patenté depuis la publication de 1897 par Ernst et Hugo Erdmann. Parmi ces auteurs, on peut citer Hagenbach Revue des colorants bleus, Helvetica Volume 6, Issue 1, 1923, pages 134-186 ; Paul Fritsch, Euric, Volume 29, Issue 2, Mai-August 1896, Pages 2290-2301. A partir des réactions de base enseignées dans ces documents, on peut produire une grande diversité de colorants, notamment des composés caractérisés par une structure triarylméthane sulfonée.

Les documents US 1531507, US7534911, US8969616, WO2017118882, WO2017218764 ,WO2018008040 rapportent différents procédés de synthèse du bleu d'isosulfan.

La plupart des méthodes de synthèse de l'art antérieur pour la fabrication de colorants triarylméthane sulfonés comprennent une étape de condensation d'un benzaldéhyde sulfoné avec la *N,N-*diéthylaniline, cette étape impliquant des acides forts et aboutissant à la formation d'une leucobase. Cette étape est généralement suivie par une étape d'oxydation au moyen d'agents oxydants connus et dont certains sont réputés dangereux (oxyde de plomb, phtalocyanine de fer / oxone), pour effectuer la conversion en colorant triarylméthane.

Le document DE46384 décrit un procédé de synthèse de colorants du type triarylméthane sulfoné, au moyen d'un procédé faisant appel à une sulfonation d'une structure triarylméthane par traitement à l'acide sulfonique fumant, suivie d'une oxydation par de l'oxyde de plomb en présence d'acide sulfurique. Ni le produit, ni sa pureté ne sont caractérisés. On ignore quelles substitutions ont été réalisées sur les cycles aromatiques. Les inventeurs de la présente demande ont constaté que le traitement par l'acide sulfonique fumant (SO₃ gazeux à saturation dans une solution concentrée de H₂SO₄) dégrade la structure triaryl méthane très rapidement. Les conditions enseignées par ce document ne permettent donc pas de former la structure visée avec des rendements et une pureté satisfaisantes.

Le document A.M. Montagut et al., Chem. Eur. J. 2017, 23, 3810-3814 décrit une synthèse de composés triarylméthane à caractère hydrophobe pour le traitement de textiles. Après la formation de la structure triarylméthane substituée par des groupements à longue chaine, la dernière étape consiste en une oxydation par une quinone, la 2,3-dichloro-5,6-dicyano-1,4-benzoquinone (DDQ). Les structures correspondantes sont significativement différentes des molécules colorantes classiques telles que le bleu patenté ou le bleu d'isosulfan dont les noyaux aryles sont substitués par des groupements à chaines courtes et qui portent des fonctions sulfonées.

Parmi les impuretés qui se forment lors de la synthèse de ces composés dialkylaminotriarylméthylsulfonés, les dérivés desalkylés sont particulièrement gênants. Dans le cas du bleu patenté et du bleu d'isosulfan, des impuretés sont désignées impuretés deséthyl.

En effet, ces molécules ne diffèrent des molécules cibles que par l'absence d'un ou de plusieurs groupements alkyle (éthyle) sur les atomes d'azote. Comme ils sont proches par leur structure des composés cibles, il est difficile de les éliminer par des techniques classiques de purification. Le document WO2017/118882 revendique la synthèse d'isosulfan blue avec une pureté >99.8% et une quantité d'impureté deséthyl <0.15%. Toutefois, le procédé décrit met en œuvre une étape d'oxydation par du permanganate de potassium qui est susceptible de conduire à une contamination par du manganèse. Cette méthode, lorsqu'elle est appliquée à la synthèse du bleu patenté, donne un composé avec une teneur en deséthyl beaucoup plus élevée.

Le document RU 2654862 décrit un procédé de purification de composés colorants diaminotriphénylméthane disulfonés. Ce procédé concerne en particulier le bleu patenté. Le procédé décrit dans ce document s'appuie sur une absorption sur un support en PDMS et une récupération du bleu patenté au moyen d'une solution aqueuse saline. Le produit issu de ce procédé est fortement chargé en sels.

Le document Journal of chromatography A 1499 (2017) 183-189 décrit un procédé de séparation électrochromatographique appliqué à des composés colorants, notamment le bleu patenté. La séparation est opérée vis-à-vis d'un mélange qui comprend deux autres colorants de structures différentes. Il n'est pas envisagé d'utiliser un tel procédé pour éliminer le composé deséthylé ou d'autres composants de structure analogue.

Il subsistait donc le besoin d'un procédé de synthèse de composés triarylméthane sulfonés qui permette d'obtenir du bleu patenté mais aussi d'autres structures telles que notamment le bleu isosulfan (isosulfan blue). En particulier, on a cherché à mettre au point un procédé qui soit reproductible, applicable à l'échelle industrielle, avec de bons rendements, et qui donne des produits d'une pureté élevée. En particulier, il subsistait le besoin d'un procédé qui produise une composition dans laquelle le composé cible est sensiblement dépourvu de dérivés désalkylés, en particulier de dérivés deséthylés dans le cas du bleu patenté et du bleu d'isosulfan, mais aussi qui ne nécessite pas l'utilisation de métaux lourds. Il subsistait aussi le besoin d'une méthode de préparation de bleu patenté qui conduise à un produit sensiblement dépourvu de sels.

De façon étonnante, la Demanderesse a découvert un procédé permettant, à partir de molécules simples et facilement disponibles, d'accéder à des composés de structure triarylméthane sulfoné, au moyen d'une étape d'oxydation par des quinones, avec des rendements et une pureté élevée. Le procédé de la divulgation donne accès à des produits qui ont à la fois une pureté organique et une pureté vis-à-vis des métaux satisfaisante, tout en ayant de bons rendements.

### Résumé de l'invention

La divulgation concerne un procédé de préparation d'un composé répondant à la formule [Chem I] 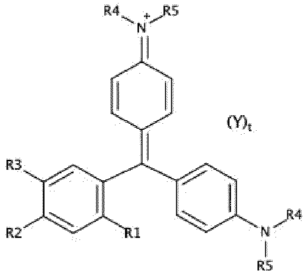 dans laquelle
R1, R2, R3 représentent, indépendamment l'un de l'autre, un groupement choisi parmi : -H, - OH, -SO₃H, -SO₃⁻, au moins l'un de R1, R2, R3 représente un groupement choisi parmi (-SO₃H, -SO₃⁻),
R4, R5, identiques ou différents, représentent un groupement choisi parmi : un alkyle en C₁-C₈, un alcényle en C₁-C₈, un phényle, un benzyle, étant entendu que deux groupements R4, R5 portés par le même atome d'azote peuvent former ensemble un cycle incluant cet atome d'azote, Y représente un cation organique ou inorganique choisi parmi les sels pharmaceutiquement acceptables ;
t représente un nombre, t = 0 ; 1/2 ; 1 ,
ce procédé étant caractérisé en ce qu'il comporte au moins une étape d'oxydation du triphénylméthane sulfoné de formule [Chem V] par une quinone choisie parmi la 1,4-benzoquinone, la 1,2-benzoquinone, une dialkyl(C₁-C₄) 1,4-benzoquinone, une dialkyl(C₁-C₄) 1,2-benzoquinone, une monoalkyl(C₁-C₄) 1,4-benzoquinone, une monoalkyl(C₁-C₄) 1,2-benzoquinone :

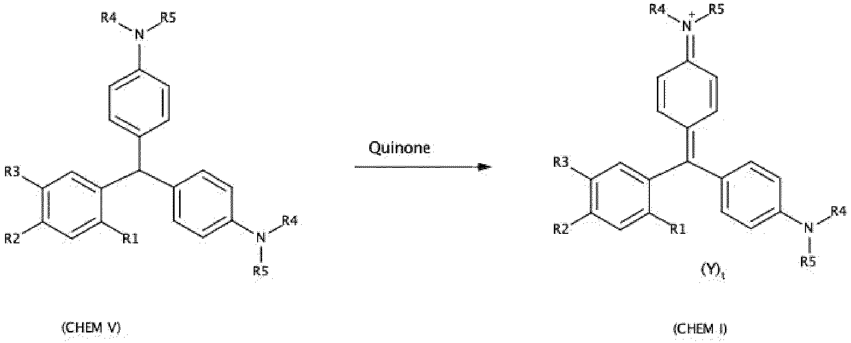

Selon un mode de réalisation préféré, la quinone est la 1,4-benzoquinone.

Selon un mode de réalisation préféré, le traitement est mis en œuvre dans un solvant protique apolaire.

Selon un mode de réalisation préféré, le traitement est mis en œuvre à une température allant de 40 à 130°C, avantageusement à une température allant de 60 à 120°C, encore plus avantageusement de 70 à 110°C.

Selon un mode de réalisation préféré, à l'issue de l'étape de traitement par une quinone, le composé cible de formule [Chem I] est séparé du milieu réactionnel par précipitation.

Selon un mode de réalisation préféré, le procédé comporte en outre une étape dans laquelle le composé cible de formule [Chem I] avec t=0 est transformé en sel de formule [Chem I] avec t=1 ou t = 1/2.

Selon un mode de réalisation préféré, le composé de formule [Chem I] répond à la formule [Chem IA]

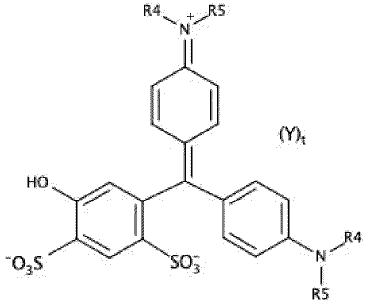

Selon un mode de réalisation préféré, le composé [Chem IA] a été obtenu par un procédé comprenant au moins les étapes suivantes :
a) la condensation du composé benzaldéhyde de formule [Chem IIA] avec la dialkylaniline de formule [Chem III] pour donner le triphénylméthane de formule [Chem IVA], 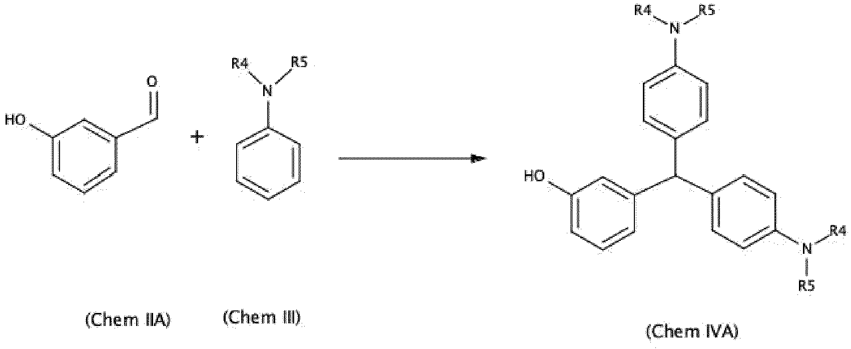
b) le traitement du triphénylméthane de formule [Chem IVA] par de l'acide sulfurique pour former le triphénylméthane sulfoné de formule [Chem VA]

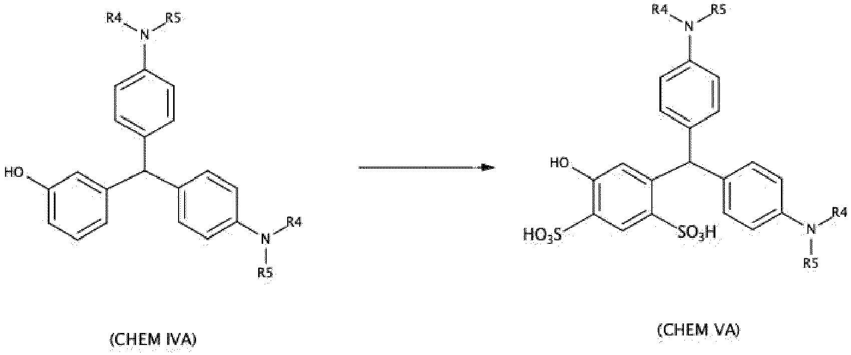

Selon un mode de réalisation encore préféré, le composé répondant à la formule [Chem I] est choisi parmi :

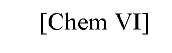

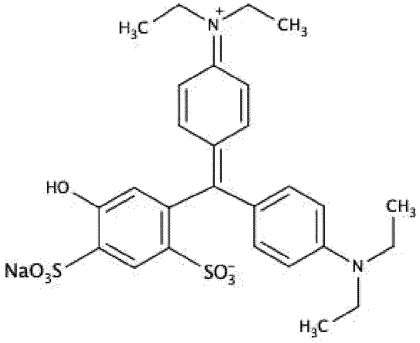

### Bleu patenté, sel de sodium

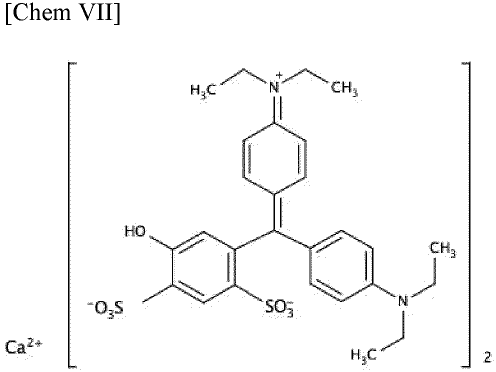

### Bleu patenté, sel de calcium

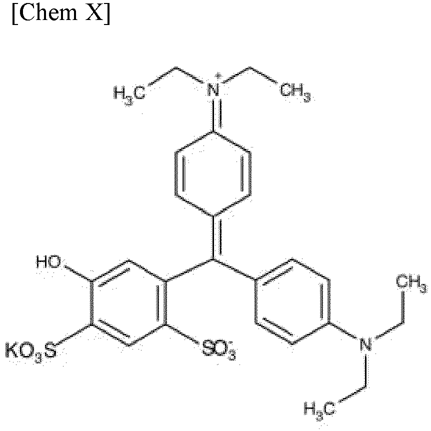

### Bleu patenté, sel de potassium

Selon une autre variante, le composé répondant à la formule [Chem I] répond à la formule [Chem IB]

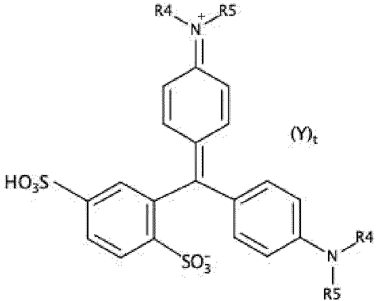

Selon un mode de réalisation préféré de cette variante, le composé répondant à la formule [Chem I] est le composé [Chem VIII]

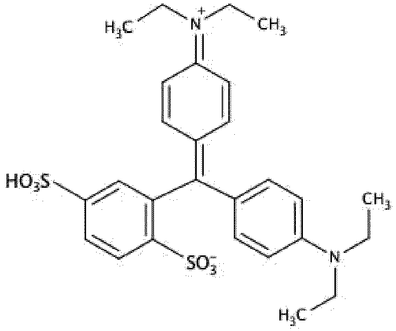

### Bleu isosulfan (Isosulfan Blue)

La divulgation concerne encore l'utilisation du procédé tel que décrit ci-dessus et de façon détaillée ci-dessous, pour obtenir une composition comprenant au moins 99,0% de composé répondant à la formule [Chem I], le % étant mesuré par chromatographie en phase liquide à haute performance avec une détection à 230 nm.

Selon un mode de réalisation préféré, l'utilisation vise à produire une composition dans laquelle aucune impureté autre qu'un dérivé monodésalkylé n'est présente en quantité supérieure à 0,1%, le % étant mesuré par chromatographie en phase liquide à haute performance avec une détection à 230 nm.

L'invention concerne encore un procédé de fabrication d'un médicament ou d'un produit de diagnostic comprenant la fabrication du composé répondant à la formule [Chem I], de préférence du bleu patenté, par le procédé tel que décrit ci-dessus et de façon détaillée ci-dessous et l'introduction du composé répondant à la formule [Chem I] dans un support pharmaceutiquement acceptable.

L'invention donne accès à une composition comprenant au moins 99,0% de composé répondant à la formule [Chem VI] ou à la formule [Chem VII], ou à la formule [Chem X], le % étant mesuré par chromatographie en phase liquide à haute performance avec une détection à 230 nm :

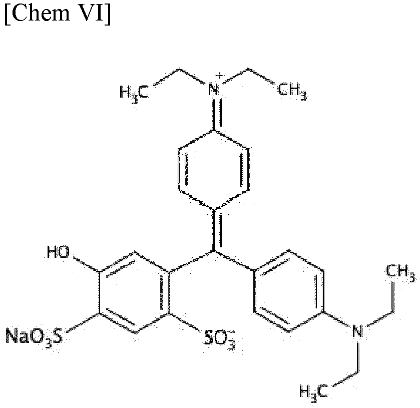

### Bleu patenté, sel de sodium

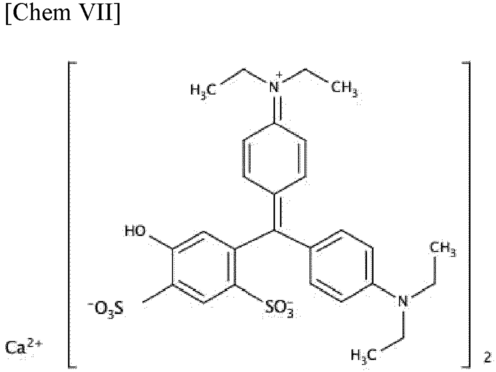

### Bleu patenté, sel de calcium.

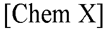

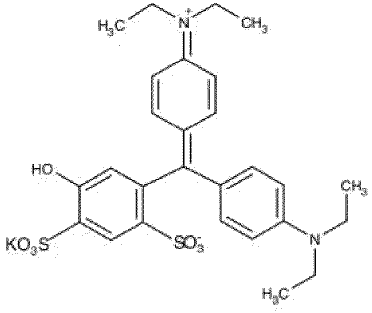

### Bleu patenté, sel de potassium

Avantageusement, dans cette composition aucune impureté autre qu'un dérivé monodésalkylé n'est présente en quantité supérieure à 0,1%, le % étant mesuré par chromatographie en phase liquide à haute performance avec une détection à 230 nm. Le procédé de la divulgation présente l'avantage de donner accès à une composition sensiblement dépourvue de NaCl, de préférence totalement dépourvue de NaCl résiduel.

L'invention concerne encore une forme cristalline du composé Bleu patenté, sel de sodium, répondant à la formule [Chem VI], caractérisée par le diagramme de diffraction X sur poudre suivant, mesuré sur un diffractomètre et exprimé en termes de distances inter-réticulaires d, d'angle de Bragg 2 thêta, d'intensité et d'intensité relative (exprimée en pourcentage par rapport à la raie la plus intense) :

| Angle 2 thêta (°) | Distance inter-réticulaire d (Å) | I (counts) | I rel (%) |
|---|---|---|---|
| 5,6 | 15,80 | 500 | 61,5 |
| 6,2 | 14,29 | 375 | 46,2 |
| 9,4 | 9,39 | 187,5 | 23,1 |
| 10,9 | 8,12 | 62,5 | 7,7 |
| 11,5 | 7,71 | 125 | 15,4 |
| 12,1 | 7,33 | 156,25 | 19,2 |
| 14,4 | 6,14 | 343,75 | 42,3 |
| 15,6 | 5,68 | 250 | 30,8 |
| 16,5 | 5,38 | 375 | 46,2 |
| 17,6 | 5,02 | 187,5 | 23,1 |
| 18,2 | 4,86 | 375 | 46,2 |
| 19,4 | 4,57 | 812,5 | 100,0 |
| 20,0 | 4,43 | 500 | 61,5 |
| 22,9 | 3,87 | 187,5 | 23,1 |
| 24,7 | 3,60 | 250 | 30,8 |

Étant entendu que les valeurs de l'intensité (I) et de l'intensité relative (I rel) des pics ci-dessus sont susceptibles de varier de +/-15%.

Avantageusement, la structure cristalline est sensiblement dépourvue de NaCl, de préférence totalement dépourvue de NaCl.

L'invention concerne la forme cristalline du composé Bleu patenté, sel de sodium, pour son utilisation comme médicament.

En particulier elle concerne la forme cristalline du composé Bleu patenté, sel de sodium, pour son utilisation dans le diagnostic, comme exposé de façon détaillée dans la description ci-dessous.

L'invention concerne encore une composition pharmaceutique comprenant au moins la forme cristalline du composé Bleu patenté, sel de sodium, répondant à la formule [Chem VI] telle que décrite ci-dessus et de façon détaillée ci-dessous, dans un support pharmaceutiquement acceptable.

De façon étonnante, on a constaté que le procédé de la divulgation donne accès directement à une forme cristalline du Bleu patenté, en particulier du Bleu patenté sel de sodium, qui n'était pas connue de l'art antérieur. L'obtention directe d'une forme cristalline dépourvue de chlorure de sodium résiduel offre de nombreux avantages en termes de rendement et de pureté, mais aussi d'efficacité et d'économie pour une application du procédé à l'échelle industrielle. Le produit obtenu présente des caractéristiques cristallines qui lui confèrent une bonne stabilité. Le procédé de la divulgation offre une plus grande facilité de façonnage dans la mesure où il conduit, de façon reproductible, à un produit cristallin, de polymorphisme unique, qui est nécessaire dans la majorité des formules galéniques (sèches en particulier). L'absence de NaCl favorise la solubilité du Bleu patenté en solution aqueuse.

### Description détaillée

L'expression « consiste essentiellement en » suivie d'une ou plusieurs caractéristiques, signifie que peuvent être inclus dans le procédé ou le matériau de l'invention, outre les composants ou étapes explicitement énumérés, des composants ou des étapes qui ne modifient pas significativement les propriétés et caractéristiques de l'invention.

L'expression « compris entre X et Y » inclut les bornes, sauf mention contraire explicite. Cette expression signifie donc que l'intervalle visé comprend les valeurs X, Y et toutes les valeurs allant de X à Y.

### Composés obtenus par le procédé de l'invention

La présente divulgation concerne un procédé d'obtention de composés répondant à la formule [Chem I] ci-dessous : 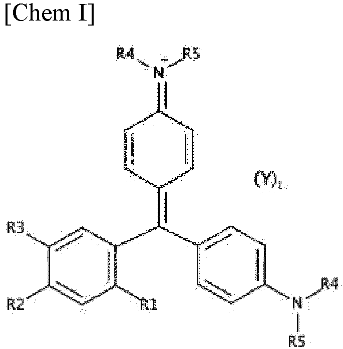 dans laquelle
R1, R2, R3 représentent, indépendamment l'un de l'autre, un groupement choisi parmi : -H, - OH, -SO₃H, -SO₃⁻,
R4, R5, identiques ou différents, représentent un groupement choisi parmi : un alkyle en C₁-C₈, un alcényle en C₁-C₈, un phényle, un benzyle, et deux groupements R4, R5 portés par le même atome d'azote peuvent former ensemble un cycle incluant cet atome d'azote,
Y représente un cation organique ou inorganique choisi parmi les sels pharmaceutiquement acceptables ;
t représente un nombre, t = 0 ; 1/2 ; 1 ;
au moins l'un de R1, R2, R3 représente un groupement choisi parmi -SO₃H, -SO₃⁻.

De préférence, au moins deux des groupements R1, R2, R3 sont choisis parmi -SO₃H, -SO₃⁻.

Avantageusement, dans la formule [Chem I] deux des groupements R1, R2, R3 sont choisis parmi -SO₃H, -SO₃⁻ et le troisième groupement est différent de -SO₃H, -SO₃⁻.

De façon préférée, dans la formule [Chem I] :
R1 = -SO₃H ou -SO₃⁻ ; R2 = H; R3 = -SO₃H ou -SO₃⁻

Ou
R1 = -SO₃H ou -SO₃⁻ ; R2 = -SO₃H ou -SO₃⁻ ; R3 = -OH.

De façon encore préférée, dans la formule [Chem I] :
R1 = -SO₃H ou -SO₃⁻ ; R2 = -SO₃H ou -SO₃⁻ ; R3 = -OH.

Lorsque deux groupements R4, R5 portés par le même atome d'azote forment ensemble un cycle, ces groupements peuvent consister en une seule chaîne alkyle ou alcényle, qui forme un cycle incluant l'atome d'azote.

Avantageusement, R4, R5, identiques ou différents, représentent un groupement choisi parmi : un alkyle en C₁-C₆, un phényle, un benzyle et deux groupements R4, R5 portés par le même atome d'azote peuvent former ensemble une chaine -(CH₂)p-, avec p un entier, p=2 à 5.

Encore plus avantageusement R4, R5, identiques ou différents, représentent un groupement choisi parmi : un alkyle en C₁-C₃, de préférence parmi le méthyle et l'éthyle. De façon préférée, (R4 ; R5) est choisi parmi (CH3 ; CH3), (C2H5 ; C2H5), (CH3 ; C2H5).

Selon un mode de réalisation préféré, R4=R5.

Encore plus avantageusement, (R4 ; R5) est choisi parmi (CH3 ; CH3) et (C2H5 ; C2H5). Selon une variante préférée, (R4 ; R5) représente (C2H5 ; C2H5).

Avantageusement, les sels pharmaceutiquement acceptables désignent des sels non toxiques dans lesquels le cation est choisi parmi les ions de métaux alcalins, de métaux alcalino-terreux, ou un ion ammonium. Encore plus avantageusement Y représente un groupement choisi parmi : Na⁺, Ca²⁺, K⁺, Mg²⁺, un groupement ammonium NH₄⁺.

De façon encore préférée, Y représente un groupement choisi parmi : Na⁺, Ca²⁺.

Selon une variante préférée, le procédé de la divulgation concerne l'obtention de composés répondant à la formule [Chem IA] ci-dessous : 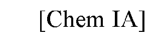 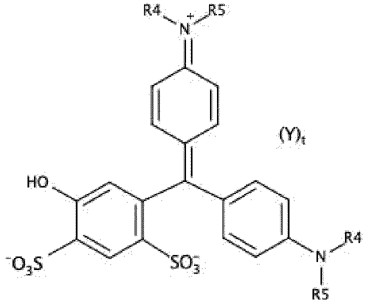 dans laquelle R4, R5, Y et t ont la même définition que dans la formule [Chem I]. Les préférences exprimées ci-dessus pour le choix de ces variables dans le contexte de la formule [Chem I] s'appliquent aussi à la formule [Chem IA].

En particulier, l'invention concerne les composés suivants :

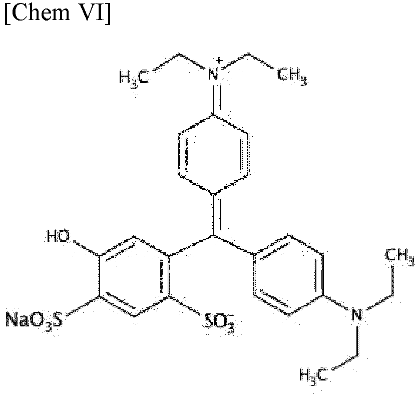

### Bleu patenté, sel de sodium

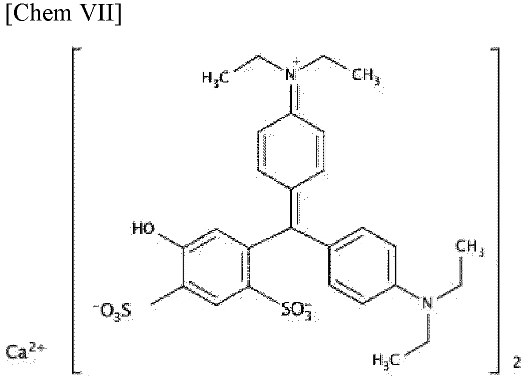

### Bleu patenté, sel de calcium

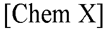

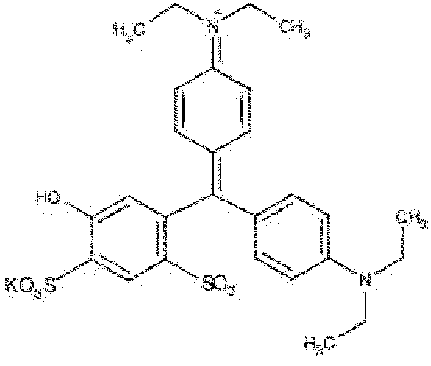

### Bleu patenté, sel de potassium

De façon encore préférée, l'invention concerne le Bleu patenté, sel de sodium et le Bleu patenté, sel de calcium.

Encore plus préférentiellement, elle concerne le Bleu patenté, sel de sodium.

Dans toute la demande, lorsque l'on mentionne le bleu patenté, on entend un composé répondant à la formule [Chem VI], à la formule [Chem VII] ou à la formule [Chem X].

Selon une autre variante, le procédé de la divulgation concerne l'obtention de composés répondant à la formule [Chem IB] ci-dessous : 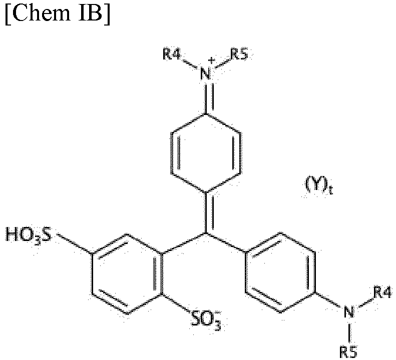 dans laquelle R4, R5, Y et t ont la même définition que dans la formule [Chem I]. Les préférences exprimées ci-dessus pour le choix de ces variables dans le contexte de la formule [Chem I] s'appliquent aussi à la formule [Chem IB].

En particulier, l'invention concerne le composé suivant :

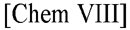

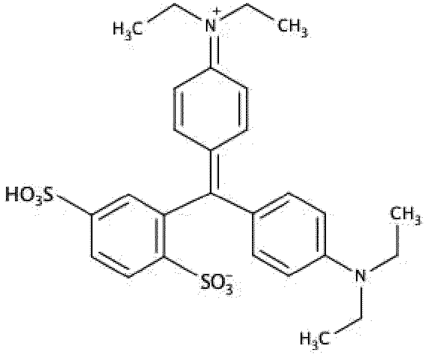

### Bleu isosulfan

Dans toute la demande, lorsque l'on mentionne le bleu d'isosulfan (Isosulfan Blue), on entend un composé répondant à la formule [Chem VIII].

Procédé de préparation d'un composé selon l'invention

### - Etape c) de traitement par une quinone :

La divulgation concerne un procédé de synthèse d'un composé répondant à la formule [Chem I] 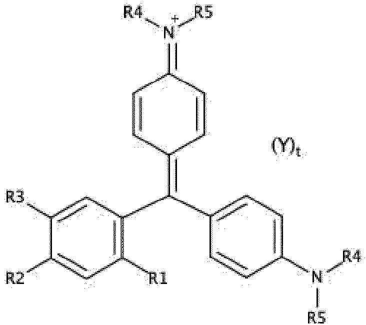 telle que définie ci-dessus, ce procédé étant caractérisé en ce qu'il comporte au moins une étape c) d'oxydation du triphénylméthane sulfoné de formule [Chem V] par une quinone choisie parmi la 1,4-benzoquinone, la 1,2-benzoquinone, une dialkyl(C₁-C₄) 1,4-benzoquinone, une dialkyl(C₁-C₄) 1,2-benzoquinone, une monoalkyl(C₁-C₄) 1,4-benzoquinone, une monoalkyl(C₁-C₄) 1,2-benzoquinone suivant le schéma :

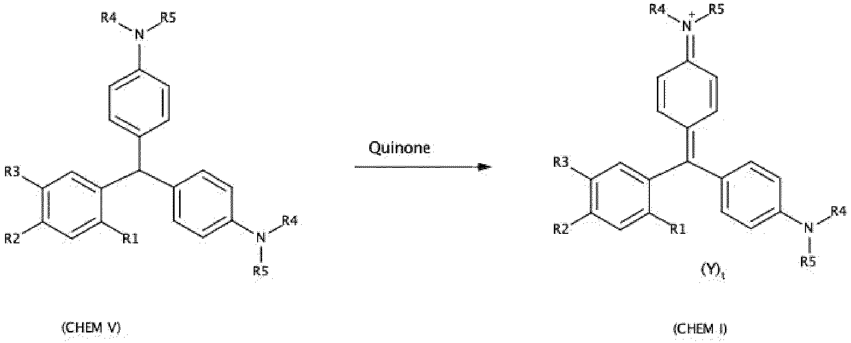

Dans la formule [Chem V], les variables R1, R2, R3, R4, R5 ont la même définition que dans la formule [Chem I] (avec les mêmes variantes préférées).

Au cours de cette étape, le triphénylméthane sulfoné de formule [Chem V] est oxydé en le composé cible de formule [Chem I] au moyen d'un traitement par une quinone. A cette étape, si l'on vise l'obtention d'un produit de haute pureté et des rendements de conversion élevés, il est important d'éviter la dégradation de la structure triarylméthane et la désalkylation, c'est-à-dire la substitution d'un ou plusieurs groupements R4, R5 par des atomes d'hydrogène.

La quinone est choisie parmi la 1,4-benzoquinone, la 1,2-benzoquinone, une dialkyl(C₁-C₄) 1,4-benzoquinone, une dialkyl(C₁-C₄) 1,2-benzoquinone, une monoalkyl(C₁-C₄) 1,4-benzoquinone, une monoalkyl(C₁-C₄) 1,2-benzoquinone.

Par dialkyl(C₁-C₄) 1,4-benzoquinone et dialkyl(C₁-C₄) 1,2-benzoquinone on entend une quinone portant deux groupements alkyle en C₁-C₄, identiques ou différents, de préférence identiques, comme par exemple la 3,5-diterbutyl-1,2-benzoquinone, la 2,5-diméthyl-1,4-benzoquinone. Par monoalkyl(C₁-C₄) 1,4-benzoquinone et monoalkyl(C₁-C₄) 1,2-benzoquinone on entend une quinone portant un groupement alkyle en C₁-C₄, comme par exemple la 2-méthyl-1,4-benzoquinone.

De préférence la quinone est la 1,4-benzoquinone.

De façon étonnante, on a constaté que ces quinones donnent un taux de conversion élevé et une faible dégradation en impuretés de type désalkyl, en particulier en impuretés deséthyl dans le cas du bleu patenté.

De préférence, à l'étape c), le traitement est mis en œuvre dans un solvant protique apolaire. Par exemple, le solvant peut être choisi parmi le diméthylsulfoxyde (DMSO), la N-méthylpyrrolidone (NMP), le diméthylformamide (DMF).

De préférence, à l'étape c), le traitement est mis en œuvre à une température allant de 40 à 130°C, avantageusement à une température allant de 60 à 120°C, encore plus avantageusement de 70 à 110°C.

Avantageusement, à l'issue de l'étape c) le composé cible de formule [Chem I] est séparé du milieu réactionnel par précipitation. De préférence, cette précipitation est provoquée par l'ajout d'un solvant ou d'un mélange de solvants choisis parmi : les alcools, les éthers, l'eau, l'acétone et leurs mélanges.

De façon alternative, il pourrait être envisagé d'isoler le composé de formule [Chem I] du milieu réactionnel par d'autres techniques de purification telles que l'extraction par des solvants ou la chromatographie sur colonne de silice.

### Étape d) de salification :

Lorsque t est différent de 0, le procédé comporte après l'étape c) une étape d) de salification. De façon bien connue, cette étape est mise en œuvre par l'introduction du composé cible de formule [Chem I] issu de l'étape c) (t=0) dans un solvant avec le contre-ion choisi.

Dans le cas particulier du bleu patenté, le contre-ion est avantageusement choisi parmi Na⁺, Ca²⁺. Mais des dérivés du bleu patenté ayant un autre contre-ion Y, correspondant au composé cible de formule [Chem IA] peuvent être obtenus par le procédé de la divulgation.

De façon optionnelle, le procédé peut être suivi d'une ou plusieurs étapes de purification classiques telles que précipitation, cristallisation, extraction par des solvants, chromatographie.

Dans le cas du composé [Chem IA], la combinaison des étapes c) et d) peut être résumée par le schéma ci-dessous :

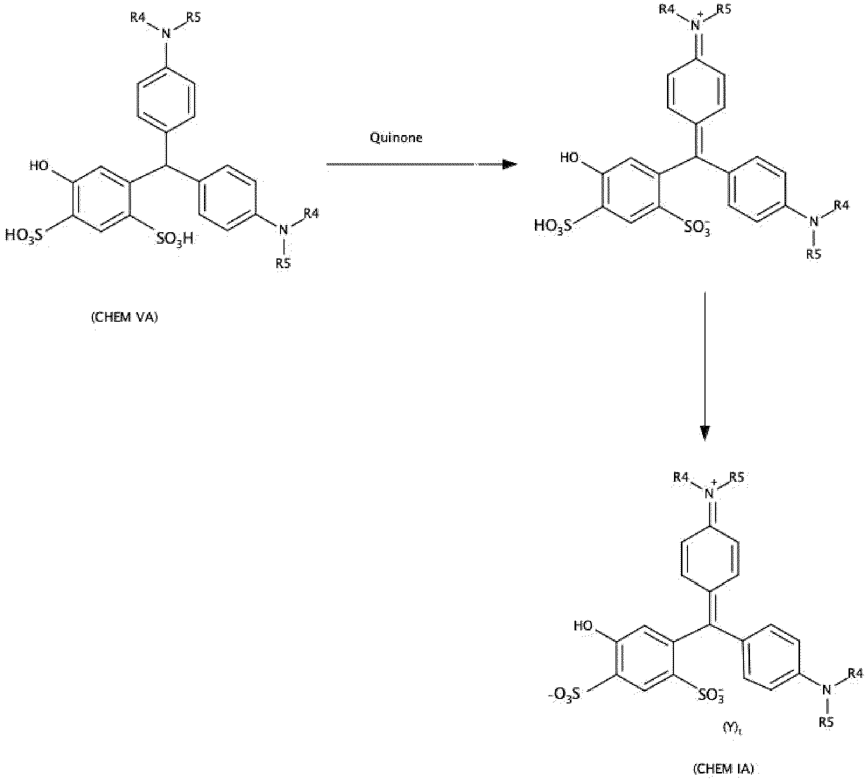

Dans le cas du Bleu patenté (sel de sodium), la combinaison des étapes c) et d) peut être résumée par le schéma ci-dessous :

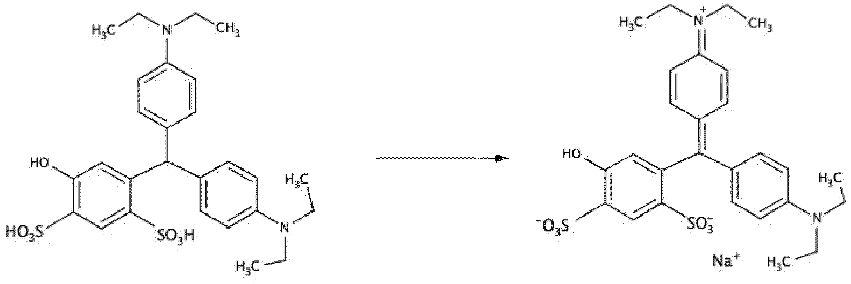

De façon étonnante, on a constaté que la combinaison des deux étapes ci-dessus donne accès directement à une forme cristalline du Bleu patenté, en particulier du Bleu patenté sel de sodium, qui n'était pas connue de l'art antérieur. L'obtention directe d'une forme cristalline offre de nombreux avantages en termes de rendement et de pureté, mais aussi d'efficacité et d'économie pour une application du procédé à l'échelle industrielle. Le produit obtenu présente des caractéristiques cristallines qui lui confèrent une bonne stabilité.

### Obtention du composé de formule [Chem V]

Pour mettre en œuvre le procédé décrit ci-dessus, on utilise un produit de formule [Chem V] qui est oxydé par une quinone dans l'étape c).

Dans certains cas, le produit de formule [Chem V] est disponible commercialement, auquel cas le procédé de la divulgation comporte une étape d'oxydation par une quinone et éventuellement une étape de salification. C'est le cas par exemple du Bleu d'isosulfan, dont le précurseur [Chem IX] ci-dessous est disponible commercialement :

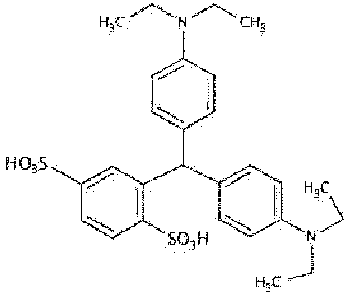

Dans d'autres cas, le précurseur de formule [Chem V] n'est pas disponible en quantité suffisante ou avec une qualité satisfaisante, auquel cas, il peut être avantageusement synthétisé par un procédé tel que décrit ci-dessous.

Le procédé de préparation du composé de formule [Chem V] comprend avantageusement au moins les étapes suivantes :
a) la condensation du composé benzaldéhyde de formule [Chem II] avec la dialkylaniline de formule [Chem III] pour donner le triphénylméthane de formule [Chem IV], 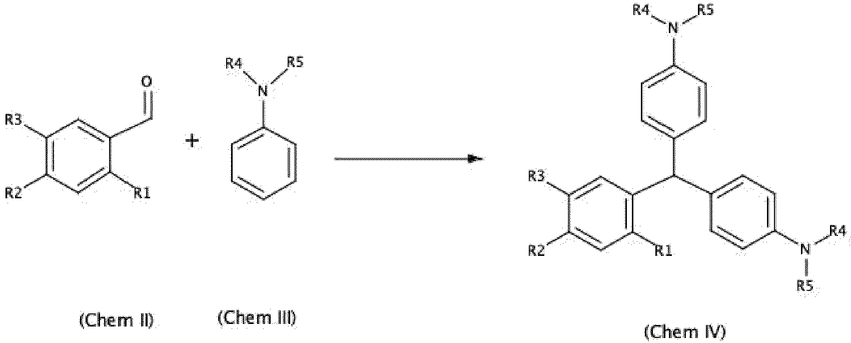 dans lesquelles
   R1, R2, R3 représentent, indépendamment l'un de l'autre, un groupement choisi parmi : -H, - OH,
   et au moins l'un de R1, R2, R3 représente H,
   R4, R5 ont la même définition que dans la formule [Chem I].
b) le traitement du triphénylméthane de formule [Chem IV] par de l'acide sulfurique pour former le triphénylméthane sulfoné de formule [Chem V]

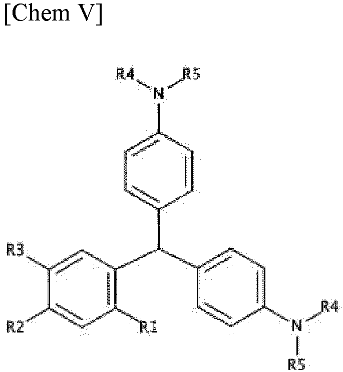

Dans la formule [Chem V], les variables R1, R2, R3, R4, R5 ont la même définition que dans la formule [Chem I] (avec les mêmes variantes préférées).

Les préférences exprimées ci-dessus pour le choix des variables R4 et R5 dans le contexte de la formule [Chem I] s'appliquent aussi aux formules [Chem II], [Chem III], [Chem IV] et [Chem V].

Les groupement R1, R2 et R3 dans les formules [Chem II] et [Chem IV] correspondent soit à R1, R2 et R3 tels que définis dans les formules [Chem I], et [Chem V], soit, pour l'un ou plusieurs de ces groupements, à un atome d'hydrogène qui est remplacé par une fonction SO₃H/SO₃⁻ au cours de l'étape b). En effet, l'étape b) opère la substitution d'un ou plusieurs atomes d'hydrogène parmi R1, R2, R3 par une fonction SO₃H/SO₃⁻.

### Étape a) :

L'étape a) consiste à condenser le composé benzaldéhyde de formule [Chem II] avec la dialkylaniline de formule [Chem III] pour donner le triphénylméthane de formule [Chem IV]. De préférence, dans les formules [Chem II] et [Chem IV], au moins deux des groupements R1, R2, R3 représentent -H.

Avantageusement, deux des groupements R1, R2, R3 sont choisis parmi H et le troisième est choisi parmi -H et -OH.

De façon préférée, dans les formules [Chem II] et [Chem IV] :
R1 = R2 = H ; R3 = -OH.

Avantageusement, dans le procédé de préparation défini ci-dessus, l'étape a) est mise en œuvre avec 1,95 à 3 équivalents molaires de dialkylaniline de formule [Chem III], par rapport à la quantité de benzaldéhyde de formule [Chem II].

Avantageusement, dans le procédé de préparation défini ci-dessus, l'étape a) est mise en œuvre en présence d'urée et en milieu acide. De préférence, le milieu réactionnel est acidifié au moyen de l'acide chlorhydrique. Avantageusement encore, le milieu réactionnel est acidifié par 1,2 à 3 équivalents molaires d'acide chlorhydrique par rapport à la quantité de benzaldéhyde de formule [Chem II]. De préférence l'étape a) est mise en œuvre en présence de 0,25 à 1,5 équivalents molaires d'urée par rapport à la quantité de benzaldéhyde de formule [Chem II]. Avantageusement, dans le procédé de préparation défini ci-dessus, l'étape a) est mise en œuvre dans un solvant choisi parmi les solvants protiques, en particulier les alcools. De préférence elle est mise en œuvre dans l'éthanol. De préférence, l'éthanol est introduit dans le milieu réactionnel en quantité allant de 1 à 6 volumes comparativement à la masse d'aldéhyde de formule [Chem II].

Avantageusement, la réaction de l'étape a) est mise en œuvre par chauffage à une température allant de 60°C à 120 °C pendant 5 à 30 heures, encore plus avantageusement à une température allant de 75°C à 100 °C pendant 10 à 25 heures.

Avantageusement, à l'issue de la réaction de condensation de l'étape a), le milieu réactionnel est traité de façon à faire précipiter le composé de formule [Chem IV]. De préférence, cette précipitation est provoquée par l'ajout dans le milieu réactionnel d'un solvant choisi parmi une solution aqueuse acide, de l'acétone, et leurs mélanges. Avantageusement, le composé de formule [Chem IV] est isolé du milieu réactionnel par filtration et purifié par lavage. Avantageusement, le lavage est fait au moyen d'un solvant choisi parmi une solution aqueuse acide, de l'acétone, et leurs mélanges.

De façon alternative, il pourrait être envisagé d'isoler le composé de formule [Chem IV] du milieu réactionnel par d'autres techniques de purification telles que l'extraction par des solvants ou la chromatographie, notamment la chromatographie sur colonne de silice.

Dans le cas où le composé [Chem I] est le composé [Chem IA], l'étape a) peut être résumée par le schéma ci-dessous :

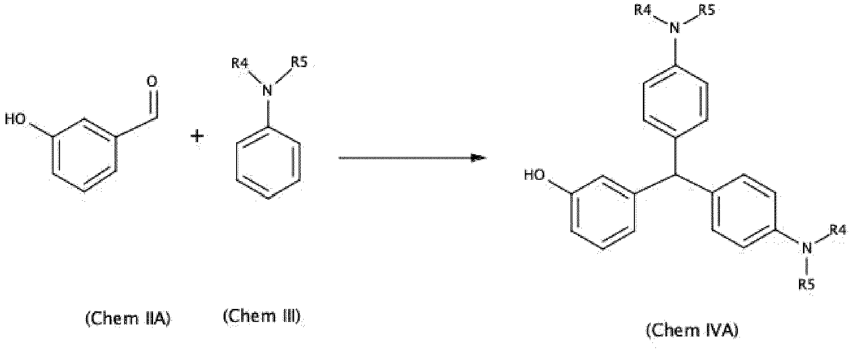

En particulier, dans le cas du Bleu patenté, l'étape a) peut être résumée par le schéma ci-dessous :

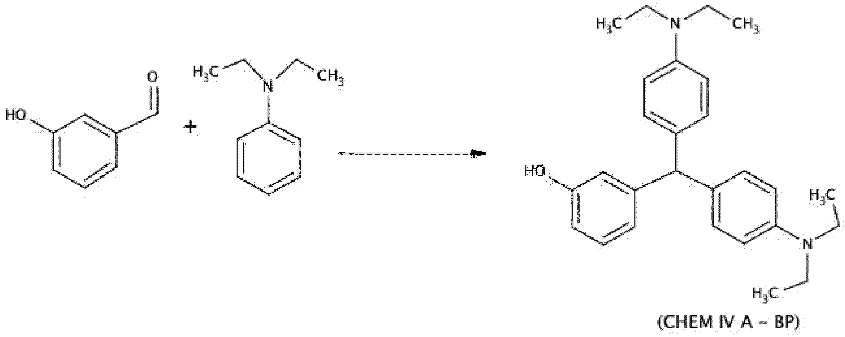

### Étape b) :

Avantageusement, dans le procédé de préparation défini ci-dessus, l'étape b) est mise en œuvre au moyen d'un traitement par de l'acide sulfurique concentré. Par exemple, on utilise une solution d'acide sulfurique de concentration supérieure ou égale à 90% volumique, de préférence une solution d'acide sulfurique de concentration supérieure ou égale à 95% volumique.

De préférence, le traitement comprend l'ajout du composé de formule [Chem IV] dans 1 à 5 volumes d'acide sulfurique.

Avantageusement, la solution du composé de formule [Chem IV] dans l'acide sulfurique est portée à une température allant de 60 à 120 °C pendant une durée allant de 30 mn à 10 h, de préférence à une température allant de 80 à 100 °C pendant une durée allant de 1 h à 5 h. Avantageusement, à l'issue de l'étape b) le triphénylméthane sulfoné de formule [Chem V] est séparé du milieu réactionnel par précipitation. De préférence, cette précipitation est provoquée par l'ajout d'un solvant ou d'un mélange de solvants choisis parmi : les alcools, les éthers, l'eau, l'acétone et leurs mélanges ; encore plus préférentiellement, par l'ajout d'alcool (éthanol, isopropanol, ...), d'un mélange eau/alcool, eau/acétone ou d'un mélange alcool/éther (isopropanol/éther isopropylique, éthanol/ éther isopropylique,...).

De façon alternative, il pourrait être envisagé d'isoler le composé de formule [Chem V] du milieu réactionnel par d'autres techniques de purification telles que l'extraction par des solvants ou la chromatographie, notamment la chromatographie sur colonne de silice.

De façon étonnante, on a constaté que dans les conditions décrites ci-dessus la sulfonation du cycle aromatique porteur de la fonction hydroxyle dans la formule [Chem IVa] se fait sélectivement en ortho et en para du cycle lorsque celui- ci comporte un groupement -OH en méta.

Dans le cas où le composé [Chem I] est le composé [Chem IA], l'étape b) peut être résumée par le schéma ci-dessous :

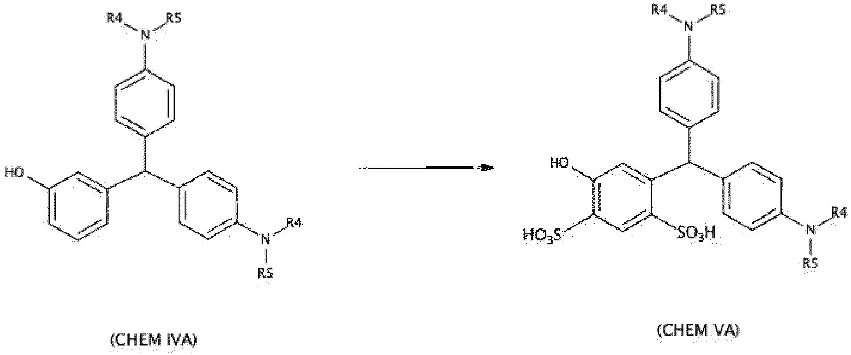

L'étape b) est donc particulièrement avantageuse pour la synthèse des composés répondant à la formule [Chem IA] qui a été définie ci-dessus. Notamment, elle permet d'obtenir du bleu patenté avec une sélectivité élevée en ce qui concerne la substitution du cycle porteur des fonctions sulfonées.

Par ailleurs, contrairement à ce qui est observé dans d'autres conditions de sulfonation, le traitement ci-dessus entraine peu de formations de produits secondaires dus à la dégradation de la structure triarylméthane ou à la désalkylation des fonctions alkylamine.

Dans le cas du Bleu patenté, l'étape b) peut être résumée par le schéma ci-dessous :

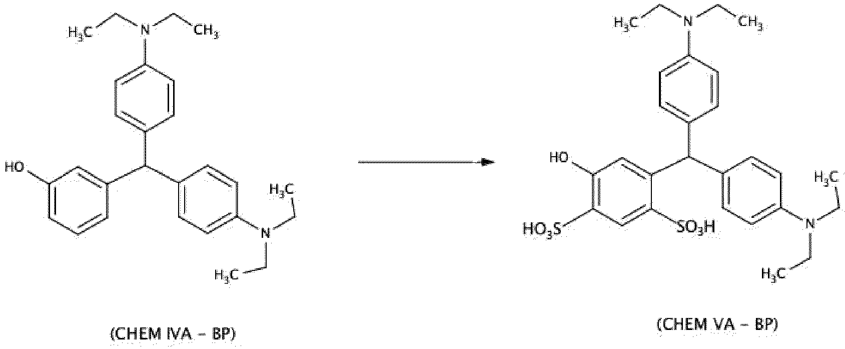

### Caractéristiques des composés obtenus

Le procédé de la divulgation donne accès à des compositions comprenant le composé cible de formule [Chem I], en particulier les composés [Chem IA] et [Chem IB], plus particulièrement les composés de formules [Chem VI], [Chem VII], [Chem X], et [Chem VIII], avec des rendements élevés et un taux d'impuretés très faible.

En pratique, l'impureté mono-desalkylée (une seule fonction alkyle parmi les groupements R4, R5 est remplacée par un atome d'hydrogène, les trois autres sont des alkyles) est la principale impureté observée lors de la synthèse des composés de formule [Chem I]. Les autres impuretés desalkylées étant de structure plus éloignée ont des propriétés différentes et, si elles se forment, elles sont éliminées au cours du procédé.

En particulier, le procédé de la divulgation donne accès à une composition comprenant au moins 98,0% de composé répondant à la formule [Chem I], et moins de 1% d'impureté monodésalkylée (composé de formule [Chem I] dans lequel un des groupements R4 et R5 représente H), le % étant mesuré par chromatographie en phase liquide à haute performance avec une détection à 230 nm.

Encore plus préférentiellement, le procédé de la divulgation donne accès à une composition comprenant au moins 99,0% de composé répondant à la formule [Chem I], et moins de 0,5% d'impureté monodésalkylée, aucune autre impureté n'étant présente en quantité supérieure à 0,15%, le % étant mesuré par chromatographie en phase liquide à haute performance avec une détection à 230 nm.

En particulier, le procédé de la divulgation donne accès à une composition comprenant au moins 98,0% de composé répondant à la formule [Chem IA] ou [Chem IB], et moins de 1% d'impureté monodésalkylée (composé dans lequel un des groupements R4, R5 représente H), le % étant mesuré par chromatographie en phase liquide à haute performance avec une détection à 230 nm. Encore plus préférentiellement, le procédé de la divulgation donne accès à une composition comprenant au moins 99,0% de composé répondant à la formule [Chem IA] ou [Chem IB], et moins de 0,5% d'impureté monodésalkylée, aucune autre impureté n'étant présente en quantité supérieure à 0,15% le % étant mesuré par chromatographie en phase liquide à haute performance avec une détection à 230 nm.

Avantageusement, le procédé donne accès à une composition dans laquelle aucune impureté autre qu'un dérivé monodesalkylé n'est présente en quantité supérieure à 0,1%, le % étant mesuré par chromatographie en phase liquide à haute performance avec une détection à 230 nm. Dans le cas du bleu patenté, les impuretés de type déséthyl sont représentées par les formules ci-dessous dans lesquelles Y et t ont la même définition que ci-dessus (en particulier Y=Na⁺ et t=1 ; Y = Ca²⁺ et t = ½ ; Y=K⁺ et t=1 ) :

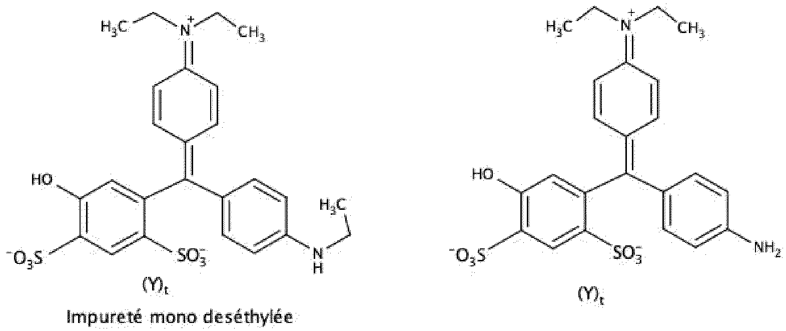

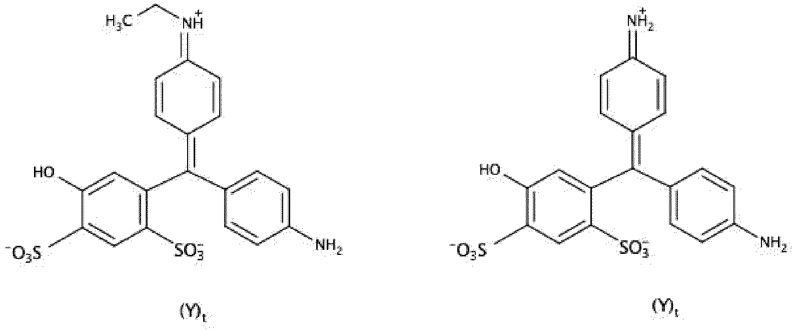

En pratique, l'impureté mono-deséthylée (une seule fonction éthyle remplacée par un atome d'hydrogène) est la principale impureté observée lors de la synthèse du bleu patenté. Les autres impuretés deséthylées étant de structure plus éloignée ont des propriétés différentes et, si elles se forment, elles sont éliminées au cours du procédé.

En particulier, le procédé de la divulgation donne accès à une composition comprenant au moins 98,0% de composé sel de 4-[[4-(diéthylamino)phényl]-(4-diéthylazaniumylidènecyclohexa-2,5-dièn-1-ylidène)méthyl]-6-hydroxybenzène-1,3-disulfonate (Bleu patenté), et moins de 1% d'impureté monodeséthylée, le % étant mesuré par chromatographie en phase liquide à haute performance avec une détection à 230 nm.

Encore plus préférentiellement, le procédé de la divulgation donne accès à une composition comprenant au moins 99,0% de composé sel de 4-[[4-(diéthylamino)phényl]-(4-diéthylazaniumylidènecyclohexa-2,5-dièn-1-ylidène)méthyl]-6-hydroxybenzène-1,3-disulfonate, et moins de 0,5% d'impureté monodeséthylée, aucune autre impureté n'étant présente en quantité supérieure à 0,15%, le % étant mesuré par chromatographie en phase liquide à haute performance avec une détection à 230 nm.

Avantageusement, le procédé donne accès à une composition dans laquelle aucune impureté autre que le dérivé monodeséthylé n'est présente en quantité supérieure à 0,1%, le % étant mesuré par chromatographie en phase liquide à haute performance avec une détection à 230 nm. En particulier, le procédé de la divulgation donne accès à une composition comprenant au moins 98,0% de composé sel de sodium du 2-[[4-(diéthylamino)phényl]-(4-diéthylazaniumylidènecyclohexa-2,5-dièn-1-ylidène)méthyl]benzène-1,4-disulfonate (Bleu d'isosulfan), et moins de 1% d'impureté monodeséthylée, le % étant mesuré par chromatographie en phase liquide à haute performance avec une détection à 230 nm.

Encore plus préférentiellement, le procédé de la divulgation donne accès à une composition comprenant au moins 99,0% de composé sel de sodium du N-[4-[[4-(diéthyl amino) phényl](2,5- disulfophényl)méthylène]-2,5-cyclohexadien-1-ylidène]-N-éthyléthanaminium, et moins de 0,5% d'impureté monodeséthylée.

Avantageusement, le procédé de la divulgation donne accès à une composition dans laquelle aucune impureté autre que le dérivé monodeséthylé n'est présente en quantité supérieure à 0,1%, le % étant mesuré par chromatographie en phase liquide à haute prerformance avec une détection à 230 nm.

Le procédé de la divulgation donne également accès à des compositions comprenant un composé répondant à la formule [Chem I], en particulier les composés [Chem IA] et [Chem IB], plus particulièrement les composés de formules [Chem VI], [Chem VII] et [Chem VIII], comportant peu ou pas d'impuretés métalliques. Il va de soi que les sels de métaux alcalins et alcalino-terreux qui peuvent correspondre à Y ne sont pas considérés comme des impuretés métalliques au sens de l'invention.

Le procédé de la divulgation donne notamment accès à des compositions comprenant un composé répondant à la formule [Chem I], en particulier les composés [Chem IA] et [Chem IB], plus particulièrement les composés de formules [Chem VI], [Chem VII] et [Chem VIII] et comportant moins de 200 ppm de contaminants métallique, avantageusement moins de 100 ppm de contaminants métalliques, encore mieux, moins de 50 ppm de contaminants métalliques, et encore plus avantageusement moins de 20 ppm de contaminants métalliques. La teneur en contaminants métalliques désigne la teneur en métaux, mesurée suivant la méthode ICP/MS (Spectrométrie de masse à plasma induit).

Par contaminants métalliques on entend tous les métaux de la classification périodique des éléments, à l'exclusion des métaux alcalins et alcalino-terreux ainsi que leurs dérivés organiques et inorganiques. Plus particulièrement, on entend par contaminants métalliques les métaux dits « lourds » et en particulier : Al, As, Cd, Cr, Cu, Fe, Sn, Mn, Hg, Mo, Ni, Pb, Zn ainsi que leurs dérivés organiques et inorganiques.

Le procédé de la divulgation donne également accès à une forme cristalline du Bleu patenté sel de sodium de formule [Chem VI], caractérisée par le diagramme de diffraction X sur poudre suivant, mesuré sur un diffractomètre (anode de cuivre) et exprimé en termes de distances inter-réticulaires d, d'angle de Bragg 2 thêta, d'intensité et d'intensité relative (exprimée en pourcentage par rapport à la raie la plus intense) :

| Angle 2 thêta (°) | Distance inter-réticulaire d (Å) | I (counts) | I rel (%) |
|---|---|---|---|
| 5,6 | 15,80 | 500 | 61,5 |
| 6,2 | 14,29 | 375 | 46,2 |
| 9,4 | 9,39 | 187,5 | 23,1 |
| 10,9 | 8,12 | 62,5 | 7,7 |
| 11,5 | 7,71 | 125 | 15,4 |
| 12,1 | 7,33 | 156,25 | 19,2 |
| 14,4 | 6,14 | 343,75 | 42,3 |
| 15,6 | 5,68 | 250 | 30,8 |
| 16,5 | 5,38 | 375 | 46,2 |
| 17,6 | 5,02 | 187,5 | 23,1 |
| 18,2 | 4,86 | 375 | 46,2 |
| 19,4 | 4,57 | 812,5 | 100,0 |
| 20,0 | 4,43 | 500 | 61,5 |
| 22,9 | 3,87 | 187,5 | 23,1 |
| 24,7 | 3,60 | 250 | 30,8 |

Il doit être entendu que les valeurs de l'intensité (I) et de l'intensité relative (I rel) des pics ci-dessus sont susceptibles de varier de +/-15%

Dans le tableau ci-dessus, toutes les raies sont supposées d'ordre 1 (n=1 dans la loi de Bragg).

### Médicament :

L'invention concerne aussi un procédé de fabrication d'un médicament ou d'un produit de diagnostic comprenant la fabrication du composé répondant à la formule [Chem I], en particulier les composés [Chem IA] et [Chem IB], plus particulièrement les composés de formules [Chem VI], [Chem VII], [Chem X] et [Chem VIII], de préférence du bleu patenté, sel de sodium, ou du bleu d'isosulfan, par le procédé décrit ci-dessus et l'introduction du composé répondant à la formule [Chem I] dans un support pharmaceutiquement acceptable.

Ledit médicament peut être sous toute forme adaptée à son utilisation dans les applications visées.

En particulier, on peut mentionner : sous forme de comprimé ou de gélule comprenant de 1 à 500 mg de composé répondant à la formule [Chem I] ; sous forme de solution aqueuse comprenant du composé répondant à la formule [Chem I], de préférence du bleu patenté ou du bleu d'isosulfan, encore plus préférentiellement le bleu patenté, sel de sodium, à une concentration allant de 0,05% à 2% en g/L. Notamment, une formulation préférée consiste en une solution aqueuse injectable.

De telles compositions comprennent, outre le composé répondant à la formule [Chem I], des excipients bien connus de l'homme du métier, tels que par exemple de l'acide citrique et/ou des citrates, un tampon phosphate, des polymères, des dérivés de cellulose, des lipides.

Dans les applications médicales, le composé répondant à la formule [Chem I], de préférence le bleu patenté ou le bleu d'isosulfan, encore plus préférentiellement le bleu patenté, lorsqu'il est obtenu par le procédé de la divulgation, présente l'avantage d'une pureté élevée, ce qui évite d'introduire dans l'organisme des matériaux sans utilité pour l'application.

L'efficacité du procédé de la divulgation permet d'accéder à un produit de pureté élevée avec des coûts réduits, facilement reproductible et applicable à l'échelle industrielle. En outre, le procédé de la divulgation permet d'accéder à un produit de structure cristalline, donc doté d'une bonne stabilité.

Une telle composition est particulièrement intéressante pour une utilisation comme agent de contraste pour le repérage des vaisseaux lymphatiques et des territoires artériels et comme agent de diagnostic du cancer. Notamment, la composition de l'invention peut être utilisée dans une procédure de diagnostic appelée « cartographie des ganglions lymphatiques sentinelles ». Cette procédure concerne tout particulièrement le cancer du sein, le cancer du côlon, le mélanome. En particulier, la composition est particulièrement intéressante pour le repérage du ganglion sentinelle avant la biopsie chez les patientes ayant un cancer du sein opérable et dans la cartographie peropératoire des ganglions lymphatiques sentinelles chez les patients atteints de cancer du côlon. Il est également utilisé dans la lymphoscintigraphie préopératoire pour la biopsie des ganglions lymphatiques sentinelles du mélanome.

Les différents modes de réalisation, variantes, les préférences et les avantages décrits ci-dessus pour chacun des objets de l'invention s'appliquent à tous les objets de l'invention et peuvent être pris séparément ou en combinaison.

L'invention est illustrée par les exemples suivants donnés à titre non limitatif.

### Figures

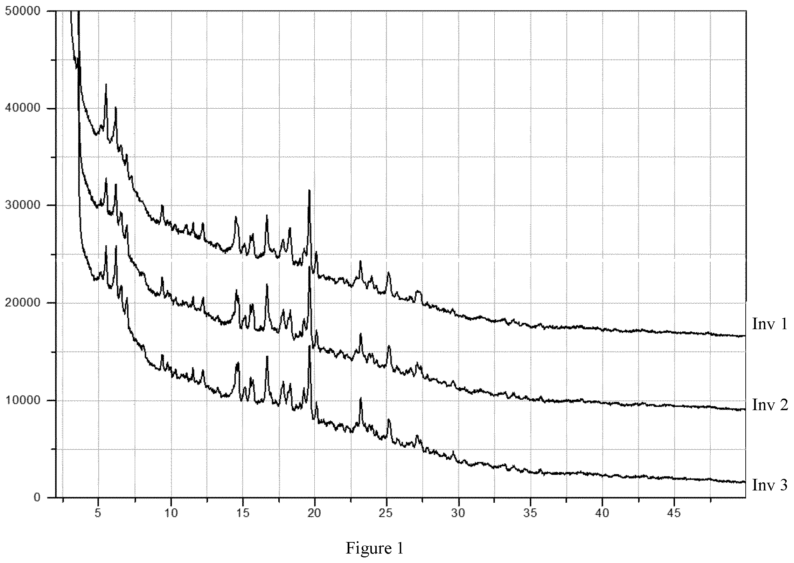

Figure 1 : la figure 1 est un graphique représentant les diagrammes de diffraction aux rayons X de trois échantillons de Bleu Patenté, sel de sodium, obtenus selon le procédé de la divulgation (désignés Inv1, Inv2 et Inv3). Il s'agit d'échantillons issus de trois lots de production distincts. En abscisse : l'angle 2 thêta en degrés ; en ordonnée : l'intensité en unité arbitraire d'intensité.

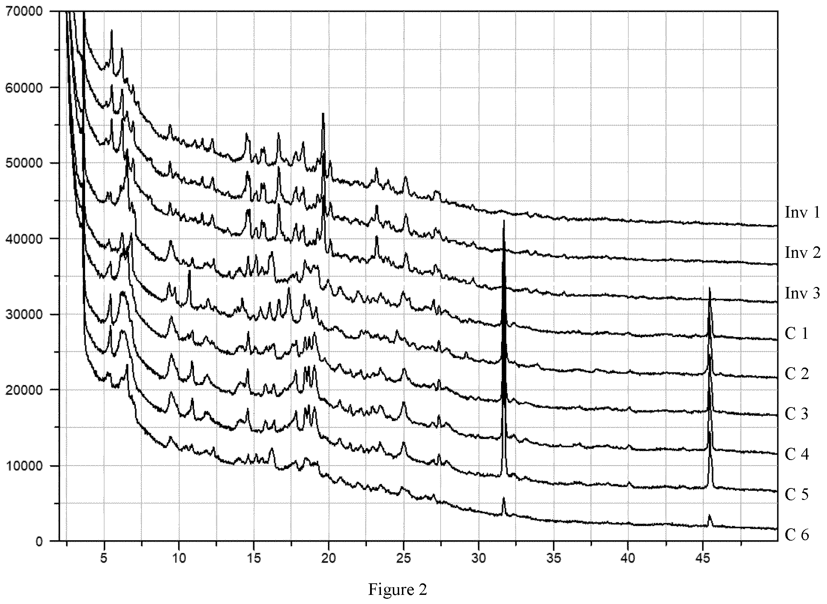

Figure 2 : la figure 2 est un graphique représentant les diagrammes de diffraction aux rayons X de six échantillons de Bleu Patenté, sel de sodium, disponibles commercialement (désignés C1 à C6). On a également reporté sur cette figure les diagrammes de diffraction aux rayons X de trois échantillons de Bleu Patenté, sel de sodium obtenus selon le procédé de la divulgation (désignés Inv1 à Inv3) et illustrés sur la figure 1. En abscisse : l'angle 2 thêta en degrés ; en ordonnée : l'intensité en unité arbitraire d'intensité.

### Exemples :

Dans ces Exemples, les parties et pourcentages sont exprimés en poids sauf indication contraire.

### Matériel et méthodes

### Analyse CLHP :

Colonne : AGILENT Poroshell 120 SB-C18 150x4,6-2,7µ (ref 683975-902)
Phase mobile A : Tampon Formiate d'Ammonium/Acide formique 10mM (pH = 4,1±0.1)
Phase mobile B : Acétonitrile
Débit : 1 ml/mn
Température : 30°C
Volume d'injection : 5µL
Gradient : Temps d'analyse 40 mn + 7mn stabilisation post run

**Tableau 1**

| Temps | A % | B % |
|---|---|---|
| 0,0 | 90 | 10 |
| 4,0 | 90 | 10 |
| 12 | 85 | 15 |
| 30 | 10 | 90 |
| 40,0 | 10 | 90 |
| 40,1 | 90 | 10 |
| 47,0 | 90 | 10 |

Détecteur : UV-Vis 230 nm et MS Electrospray (mode négatif)
Diagramme de diffraction X sur poudre : Le diagramme de diffraction X sur poudre a été réalisé avec les conditions expérimentales suivantes :
- Diffractomètre X'Pert Pro MPD Panalytical (DY2764),
- Anode de cuivre (λ=1,54Å), voltage : 40 kV, intensité 40 mA
- Montage θ-θ
- Domaine de mesure : 2° à 50 °
- Incrémentation entre chaque mesure : 0.026 °
- Temps de mesure par pas : 20.40 s,
- Détecteur PIXcel RTMS (PHD 25.5-7%, active length 3.347 °

### Synthèse du bleu patenté (selon l'invention) :

### Étape 1 : Préparation du dichlorhydrate de 3-(bis(4-(diéthylamino)phenyl)methyl)phénol :

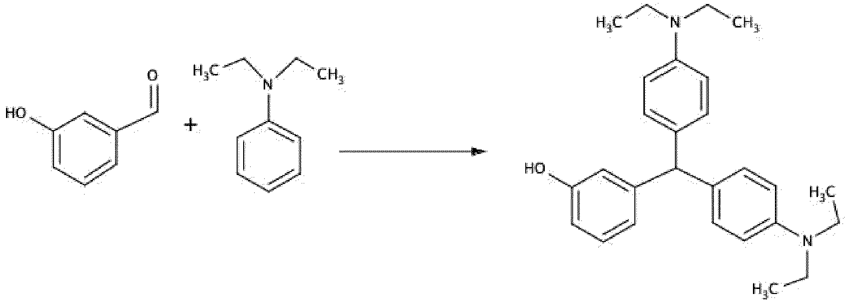

Ajouter la *N,N*-diéthylaniline (254.0 mL, 1.597 mol, 1.95 eq) à un mélange de 3-hydroxybenzaldéhyde (100 g, 0.819 mol, 1.00 eq) et d'urée (24.6 g, 0.409 mol, 0.50 eq) dans 100 mL d'éthanol.

Refroidir le milieu réactionnel à 0°C et couler une solution aqueuse de HCl 37% (136.5 mL, 1.638 mol, 2.00 eq) en maintenant le milieu à une température inférieure à 20°C.

Ensuite chauffer le milieu réactionnel au reflux pendant 20h.

Refroidir le milieu réactionnel à 60° C puis couler 800mL d'une solution aqueuse de HCl 2N. Refroidir le milieu réactionnel à 0°C et maintenir sous agitation pendant 5 heures.

Filtrer le précipité obtenu et laver le solide avec 500 mL d'un mélange acétone/HCl aqueux 2N (9/1) puis 500 mL d'acétone.

Le solide est séché à l'étuve ventilée (50°C).

On obtient 303 g de dichlorhydrate de 3-(bis(4-(diéthylamino)phényl)méthyl)phénol sous forme de solide blanc.
Pureté CLHP : 97.2%
Rendement : 78%
¹H RMN (300 MHz, D2O) : δ 7.39 (4H, dd, *J*=8.7 Hz), 7.33 (4H, dd, *J*=8.7 Hz), 7.22 (1H, t, *J*=7.9 Hz), 6.80 (1H, ddd), 6.71 (1H, dd, *J*=7.8 Hz), 6.67 (1H, t, *J*=2.0 Hz), 5.67 (1H, s), 3.60 (8H, q, *J*=6.9 Hz), 1.06 (12H, t, *J*=7.2 Hz)
¹³C RMN (75 MHz, D2O) : δ 156.0, 145.5, 144.3, 135.1, 131.2, 130.3, 122.4, 121.4, 116.2, 114.0, 54.9, 53.7, 9.7

### Étape 2: Préparation de l'acide 4-(bis(4-(diéthylamino)phényl)méthyl)-6-hydroxybenzène-1,3-disulfonique :

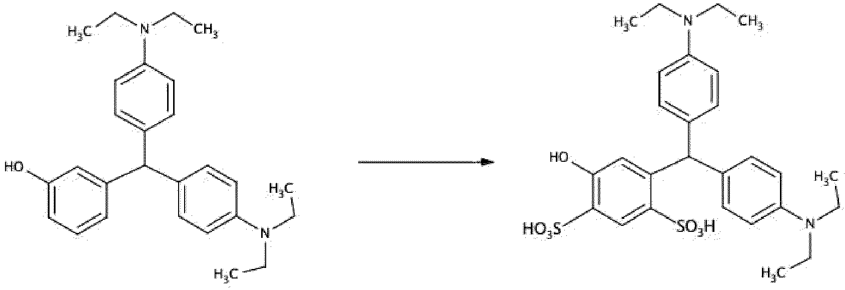

Ajouter le dichlorhydrate de 3-(bis(4-(diéthylamino)phényl)méthyl)phénol (150 g, 0.315 mol, 1 eq.) dans 600 mL d'H₂SO₄.

Chauffer à 90°C pendant 3h puis laisser 16h sous agitation à température ambiante.

Diluer le milieu réactionnel avec 15 L d'un mélange éthanol/éther isopropylique (75/25) en maintenant une température inférieure à 20°C.

Filtrer le précipité obtenu et laver le solide avec 2x600 mL d'éther isopropylique.

Le solide est séché à l'étuve ventilée (50°C).

On obtient 183 g d'acide 4-(bis(4-(diéthylamino)phényl)méthyl)-6-hydroxybenzène-1,3-disulfonique sous forme de solide blanc.
Pureté CLHP : 98.8%
Rendement : quantitatif
¹H RMN (300 MHz, DMSO) : δ 10.91 (2H, br s), 8.08 (1H, s), 7.52 (4H, dd, *J*=8.5 Hz), 7.38 (4H, dd, *J*=8.6 Hz), 6.95 (1H, s), 6.54 (1H, s), 4.75 (1H, br s), 3.54 (8H, q, *J*=7.1 Hz), 0.92 (12H, t, *J*=6.7 Hz)
¹³C RMN (75 MHz, DMSO) : δ 153.6, 145.2, 143.0, 137.1, 135.5, 130.8, 128.0, 126.7, 122.3, 118.4, 52.5, 49.2, 10.1

### Étape 3: Préparation du Bleu patenté :

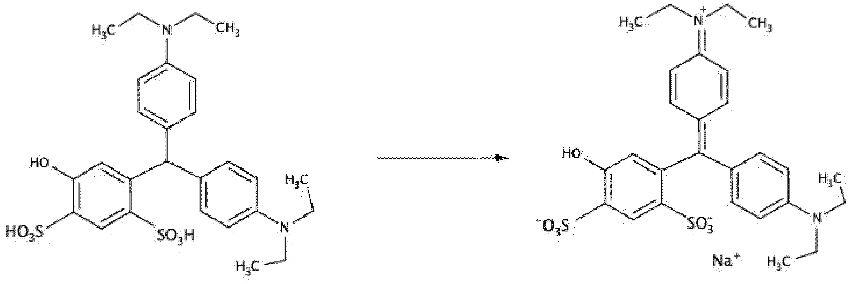

Ajouter l'acide 4-(bis(4-(diéthylamino)phényl)méthyl)-6-hydroxybenzène-1,3-disulfonique (1.2 kg, 2.1 mol, 1 eq.) dans 6 L de N-méthylpyrrolidone.

Chauffer le milieu réactionnel à 95°C et couler la 1,4-benzoquinone (346 g, 3.2 mol, 1.5 eq.) en solution dans 1.2 L de N-méthylpyrrolidone.

Poursuivre le chauffage pendant 3 heures puis redescendre à température ambiante.

Diluer le milieu réactionnel avec 18 L d'acétone.

Filtrer le précipité obtenu et laver le solide avec 2x 6 L d'acétone.

Reprendre le solide dans 6 L de mélange H₂O/Methanol (80/20).

Chauffer 1h au reflux puis redescendre à température ambiante.

Filtrer le solide et laver avec 2x6 L d'acétone. Reprendre le solide dans 15 L de méthanol sous agitation et couler une solution aqueuse de sodium carbonate 22% (256 mL).

Laisser sous agitation pendant 3 heures puis concentrer.

Reprendre dans 10 L d'isopropanol.

Récupérer le solide par filtration et laver avec 2x4 L d'acétone

Le solide est séché à l'étuve ventilée (50°C).

On obtient 766 g de sel de sodium de 4-[[4-(diéthylamino)phényl]-(4-diéthylazaniumylidènecyclohexa-2,5-dièn-1-ylidène)méthyl]-6-hydroxybenzène-1,3-disulfonate (Bleu patenté) sous forme de solide violet.
Pureté CLHP: 99.3 %
Rendement : 61 %
¹H RMN (300 MHz, D2O) : δ 8.29 (1H, s), 7.24 (4H, dd, *J*=8.8 Hz), 6.72 (4H, dd, *J*=8.9 Hz), 6.41 (1H, s), 3.49 (8H, q, *J*=5.8 Hz), 1.12 (12H, t, *J*=5.9 Hz)
¹³C RMN (75 MHz, D2O) : δ 170.1, 156.6, 154.9, 141.6, 139.7, 133.2, 128.9, 128.2, 125.6, 121.2, 113.5, 45.8, 12.2

### Synthèse du bleu patenté (comparatif) :

On a procédé comme décrit ci-dessus pour les étapes 1 et 2. Pour l'étape 3, on a suivi le protocole décrit ci-dessus en faisant varier le composé oxydant. Ce composé oxydant pouvait être une autre quinone ou un autre réactif oxydant.

Les résultats obtenus sont rapportés dans les tableaux 2 et 3 ci-dessous

**Tableau 2**

| Quinone | Taux de conversion étape 3 (*) | Composé mono deséthylé % (*) |
|---|---|---|
| 1,4-benzoquinone (a) | 93% | <1% |
| 3,5-Diterbutyl-1,2-benzoquinone (a) | 93% | 2,6% |
| 2,5-Diméthyl-1,4-benzoquinone (a) | 70% | 1,7% |
| 2-Méthyl-1,4-benzoquinone (a) | 52% | 2,1% |
| 2,3-Dichloro-5,6-dicyano-1,4-benzoquinone (b) | 31% | 2,4% |
| Tétrachloro-1,4-benzoquinone (b) | 90% | 6,8% |
| Tétrachloro-1,2-benzoquinone (b) | 65% | 4% |
| 2-Chloro-1,4-benzoquinone (b) | 92% | 3,7% |

| | | |
|---|---|---|
| (*) mesuré selon la méthode CLHP décrite ci-dessus (a) selon l'invention (b) comparatif | | |

On constate que certaines quinones donnent accès à des taux de conversion élevés tout en conduisant à un produit comprenant peu d'impureté monodeséthylée. D'autres quinones donnent soient des rendements très faibles, soit une teneur élevée en composé monodeséthylé, difficile à séparer.

**Tableau 3**

| Oxydant (comparatif) | Taux de conversion étape 3 (*) | Composé mono deséthylé % (*) |
|---|---|---|
| KMnO₄ | 95% | 5% |
| NaMnO₄ | 78% | 7% |
| Ammonium Cerium (IV) Nitrate | 41% | 4% |
| MnO₂ | 38% | 5% |
| FeCl₃ | - | - |

| | | |
|---|---|---|
| (*) mesuré selon la méthode CLHP décrite ci-dessus | | |

On constate que seuls quelques réactifs oxydants autres que des quinones permettent de transformer le composé (V) en bleu patenté. Rares sont ceux qui permettent d'obtenir une teneur en deséthyl inférieure à 5 %, ou avec un taux de conversion peu élevé. En outre, ces réactifs sont à base de métaux lourds, dont l'utilisation est peu recommandée, aussi bien pour des raisons écologiques que si l'on souhaite éviter une contamination du produit final.

### Diffractogramme de rayons X sur poudre :

Le profil de diffraction des rayons X de la poudre (angles de diffraction) du Bleu patenté sel de sodium obtenu à l'exemple 3 est donné par les raies significatives rapportées dans le tableau 4 avec leur intensité et leur intensité relative (exprimée en pourcentage par rapport à la raie la plus intense. La mesure a été réalisée sur trois lots distincts qui présentent tous sensiblement le même profil comme représenté sur la figure 1. Les lots Inv 1, Inv 2 et Inv 3 ont été préparés à l'échelle pilote (quantité préparée 3 à 6 kg). Cette mesure confirme que les caractéristiques cristallines du produit sont constantes à l'issue du procédé de préparation. Elle confirme également l'absence de NaCl (voir pics caractéristiques sur la figure 2).

**Tableau 4**

| Angle 2 thêta (°) | Distance inter-réticulaire d (Å) | I (counts) | I rel (%) |
|---|---|---|---|
| 5,6 | 15,80 | 500 | 61,5 |
| 6,2 | 14,29 | 375 | 46,2 |
| 9,4 | 9,39 | 187,5 | 23,1 |
| 10,9 | 8,12 | 62,5 | 7,7 |
| 11,5 | 7,71 | 125 | 15,4 |
| 12,1 | 7,33 | 156,25 | 19,2 |
| 14,4 | 6,14 | 343,75 | 42,3 |
| 15,6 | 5,68 | 250 | 30,8 |
| 16,5 | 5,38 | 375 | 46,2 |
| 17,6 | 5,02 | 187,5 | 23,1 |
| 18,2 | 4,86 | 375 | 46,2 |
| 19,4 | 4,57 | 812,5 | 100,0 |
| 20,0 | 4,43 | 500 | 61,5 |
| 22,9 | 3,87 | 187,5 | 23,1 |
| 24,7 | 3,60 | 250 | 30,8 |

### Comparaison avec des produit Bleu patenté, sel de sodium du commerce :

Les produits commerciaux des producteurs suivants (Tableau 5) ont été analysés par la même méthode de diffraction X :

**Tableau 5 : Bleu patenté, sel de sodium du commerce**

| Producteur | Référence commerciale | Référence sur la figure 2 |
|---|---|---|
| ACROS | 339330050 | C1 |
| Santa Cruz Biotechnology | SC250653 | C2 |
| TCI | A1242 | C3 |
| Combi Blocks | HA8936 | C4 |
| Biosynth | FC1571 | C5 |
| Colorey | FG18191327 | C6 |

Les résultats sont rapportés sur la figure 2. On constate que la plupart des pics ne correspondent pas à ceux du Bleu patenté, sel de sodium, de l'invention qui sont rapportés dans le tableau 4 et sur la figure 1. Ce constat permet de conclure que le Bleu patenté, sel de sodium, de l'invention est une forme cristalline nouvelle par rapport aux formes cristallines déjà connues. Sur la figure 2 on observe également la présence de pics caractéristiques de NaCl, notamment les pics aux angles 2 thêta = 32 et 45,5, ce qui permet de constater la présence de NaCl dans tous les échantillons de Bleu patenté du commerce, mais pas dans le Bleu patenté sel de sodium selon l'invention.

## Revendications

1. Composition comprenant au moins 98% de bleu patenté sel de sodium, et moins de 1% d'impuretés monodeséthylées, le pourcentage étant mesuré par chromatographie en phase liquide à haute performance avec une détection à 230 nm, **caractérisée en ce que** le bleu patenté sel de sodium est sous forme cristalline.

2. Composition selon la revendication 1 où la forme cristalline du bleu patenté sel de sodium est **caractérisée par** un diagramme de diffraction X sur poudre comprenant les pics suivants :
| Angle 2 thêta (°) |
|---|
| 5.6 |
| 6.2 |
| 9.4 |
| 10,9 |
| 11,5 |
| 12,1 |
| 14,4 |
| 15,6 |
| 16,5 |
| 17,6 |
| 18,2 |
| 19,4 |
| 20,0 |
| 22,9 |
| 24,7 |

3. Composition selon la revendication 2 où le diagramme de diffraction X sur poudre est en outre **caractérisé par** les distances inter-réticulaires d, les intensités et les intensités relatives suivantes :
| Angle 2 thêta (°) | Distance inter-réticulaire d (Å) | I (counts) | I rel (%) |
|---|---|---|---|
| 5,6 | 15,80 | 500 | 61,5 |
| 6,2 | 14,29 | 375 | 46,2 |
| 9,4 | 9,39 | 187,5 | 23,1 |
| 10,9 | 8,12 | 62,5 | 7,7 |
| 11,5 | 7,71 | 125 | 15,4 |
| 12,1 | 7,33 | 156,25 | 19,2 |
| 14,4 | 6,14 | 343,75 | 42,3 |
| 15,6 | 5,68 | 250 | 30,8 |
| 16,5 | 5,38 | 375 | 46,2 |
| 17,6 | 5,02 | 187,5 | 23,1 |
| 18,2 | 4,86 | 375 | 46,2 |
| 19,4 | 4,57 | 812,5 | 100,0 |
| 20,0 | 4,43 | 500 | 61,5 |
| 22,9 | 3,87 | 187,5 | 23,1 |
| 24,7 | 3,60 | 250 | 30,8 |
les valeurs de l'intensité (I) et de l'intensité relative (I rel) des pics étant susceptibles de varier de +/-15%.

4. Composition selon l'une quelconque des revendications 1 à 3, comprenant au moins 99% de bleu patenté sel de sodium, et moins de 0,5% d'impuretés monodeséthylée, le pourcentage étant mesuré par chromatographie en phase liquide à haute performance avec une détection à 230 nm.

5. Composition selon l'une quelconque des revendications 1 à 4, où aucune impureté autre que les impuretés monodeséthylées n'est présente en quantité supérieure à 0,15%, le pourcentage étant mesuré par chromatographie en phase liquide à haute performance avec une détection à 230 nm.

6. Composition selon la revendication 5, où aucune impureté autre que les impuretés monodeséthylées n'est présente en quantité supérieure à 0,1%, le pourcentage étant mesuré par chromatographie en phase liquide à haute performance avec une détection à 230 nm.

7. Composition selon l'une quelconque des revendications 1 à 6, comprenant moins de 200 ppm de contaminants métalliques, avantageusement moins de 100 ppm de contaminants métalliques, encore mieux, moins de 50 ppm de contaminants métalliques, et encore plus avantageusement moins de 20 ppm de contaminants métalliques.

8. Composition selon l'une quelconque des revendications 1 à 7 qui est dépourvue de NaCl.

9. Composition selon l'une quelconque des revendications 1 à 8 pour son utilisation comme médicament.

10. Composition selon l'une quelconque des revendications 1 à 8 pour son utilisation comme produit de diagnostic.

11. Composition selon l'une quelconque des revendications 1 à 8 pour son utilisation comme agent de contraste dans le repérage des vaisseaux lymphatiques et des territoires artériels et/ou comme agent de diagnostic du cancer.

12. Composition selon l'une quelconque des revendications 1 à 8 pour son utilisation selon la revendication 11 dans le repérage du ganglion sentinelle avant la biopsie chez les patientes atteint d'un cancer du sein opérable ou dans la cartographie peropératoire des ganglions lymphatiques sentinelles chez les patients atteints d'un cancer du côlon.

13. Procédé de fabrication d'un médicament ou d'un produit de diagnostic comprenant la fabrication de la composition selon l'une quelconque des revendications 1 à 8 et l'introduction de ladite composition dans un support pharmaceutiquement acceptable.

14. Composition sous forme de comprimé ou de gélule comprenant de 1 à 500 mg d'une composition selon l'une quelconque des revendications 1 à 8 dans un support pharmaceutiquement acceptable.

## Patentansprüche

1. Zusammensetzung, umfassend mindestens 98 % Natriumsalzpatentblau und weniger als 1 % monodesethylierte Verunreinigungen, wobei der Prozentsatz durch Hochleistungsflüssigkeitschromatographie mit einer Detektion bei 230 nm gemessen wird, **dadurch gekennzeichnet, dass** das Natriumsalzpatentblau in kristalliner Form ist.

2. Zusammensetzung nach Anspruch 1, wobei die kristalline Form von Natriumsalzpatentblau durch ein Röntgenpulverbeugungsdiagramm gekennzeichnet ist, umfassend die folgenden Peaks:
| Winkel 2 Theta (°) |
|---|
| 5,6 |
| 6,2 |
| 9,4 |
| 10,9 |
| 11,5 |
| 12,1 |
| 14,4 |
| 15,6 |
| 16,5 |
| 17,6 |
| 18,2 |
| 19,4 |
| 20,0 |
| 22,9 |
| 24,7 |

3. Zusammensetzung nach Anspruch 2, wobei das Röntgenpulverbeugungsdiagramm ferner durch die folgenden Zwischengitterabstände d, Intensitäten und relativen Intensitäten gekennzeichnet ist:
| Winkel 2 Theta (°) | Zwischengitterabst and d (Å) | I (Zählung) | I rel. (%) |
|---|---|---|---|
| 5,6 | 15,80 | 500 | 61,5 |
| 6,2 | 14,29 | 375 | 46,2 |
| 9,4 | 9,39 | 187,5 | 23,1 |
| 10,9 | 8,12 | 62,5 | 7,7 |
| 11,5 | 7,71 | 125 | 15,4 |
| 12,1 | 7,33 | 156,25 | 19,2 |
| 14,4 | 6,14 | 343,75 | 42,3 |
| 15,6 | 5,68 | 250 | 30,8 |
| 16,5 | 5,38 | 375 | 46,2 |
| 17,6 | 5,02 | 187,5 | 23,1 |
| 18,2 | 4,86 | 375 | 46,2 |
| 19,4 | 4,57 | 812,5 | 100,0 |
| 20,0 | 4,43 | 500 | 61,5 |
| 22,9 | 3,87 | 187,5 | 23,1 |
| 24,7 | 3,60 | 250 | 30,8 |
wobei die Werte für die Intensität (I) und die relative Intensität (I rel.) der Peaks um +/-15 % variieren können.

4. Zusammensetzung nach einem der Ansprüche 1 bis 3, umfassend mindestens 99 % Natriumsalzpatentblau und weniger als 0,5 % monodesethylierte Verunreinigungen, wobei der Prozentsatz durch Hochleistungsflüssigkeitschromatographie mit einer Detektion bei 230 nm gemessen wird.

5. Zusammensetzung nach einem der Ansprüche 1 bis 4, wobei keine anderen Verunreinigungen als die monodesethylierten Verunreinigungen in einer Menge von mehr als 0,15 % vorhanden sind, wobei der Prozentsatz durch Hochleistungsflüssigkeitschromatographie mit einer Detektion bei 230 nm gemessen wird.

6. Zusammensetzung nach Anspruch 5, wobei keine anderen Verunreinigungen als die monodesethylierten Verunreinigungen in einer Menge von mehr als 0,1 % vorhanden sind, wobei der Prozentsatz durch Hochleistungsflüssigkeitschromatographie mit einer Detektion bei 230 nm gemessen wird.

7. Zusammensetzung nach einem der Ansprüche 1 bis 6, umfassend weniger als 200 ppm Metallverunreinigungen, vorteilhafterweise weniger als 100 ppm Metallverunreinigungen, noch besser weniger als 50 ppm Metallverunreinigungen und noch vorteilhafterweise weniger als 20 ppm Metallverunreinigungen.

8. Zusammensetzung nach einem der Ansprüche 1 bis 7, die frei von NaCl ist.

9. Zusammensetzung nach einem der Ansprüche 1 bis 8 zur Verwendung als Medikament.

10. Zusammensetzung nach einem der Ansprüche 1 bis 8 zur Verwendung als Diagnostikprodukt.

11. Zusammensetzung nach einem der Ansprüche 1 bis 8 zur Verwendung als Kontrastmittel bei der Markierung von Lymphgefäßen und arteriellen Territorien und/oder als Mittel für Krebsdiagnose.

12. Zusammensetzung nach einem der Ansprüche 1 bis 8 zur Verwendung nach Anspruch 11 bei der Sentinel-Lymphknoten-Markierung vor der Biopsie bei Patientinnen mit operablem Brustkrebs oder bei der intraoperativen Kartierung von Sentinel-Lymphknoten bei Patienten mit Dickdarmkrebs.

13. Verfahren zur Herstellung eines Medikaments oder Diagnostikprodukts, umfassend die Herstellung der Zusammensetzung nach einem der Ansprüche 1 bis 8 und das Einbringen der Zusammensetzung in einen pharmazeutisch annehmbaren Träger.

14. Zusammensetzung in Form einer Tablette oder einer Kapsel, umfassend 1 bis 500 mg einer Zusammensetzung nach einem der Ansprüche 1 bis 8 in einem pharmazeutisch annehmbaren Träger.

## Claims

1. A composition comprising at least 98 % patent blue sodium salt and less than 1 % mono-desethyl impurities, the percentage being measured by high performance liquid chromatography with detection at 230 nm, **characterised in that** the patent blue sodium salt is in crystalline form.

2. The composition according to claim 1, wherein the crystalline form of the patent blue sodium salt is **characterised by** an X-ray powder diffraction pattern having the following peaks:
| 2Theta angle (°) |
|---|
| 5.6 |
| 6.2 |
| 9.4 |
| 10.9 |
| 11.5 |
| 12.1 |
| 14.4 |
| 15.6 |
| 16.5 |
| 17.6 |
| 18.2 |
| 19.4 |
| 20.0 |
| 22.9 |
| 24.7 |

3. The composition according to claim 2, wherein the X-ray powder diffraction pattern is additionally **characterised by** the following inter-reticular distances d, intensities and relative intensities:
| 2Theta angle (°) | Inter-reticular distance d (Å) | I (counts) | rel I (%) |
|---|---|---|---|
| 5.6 | 15.80 | 500 | 61.5 |
| 6.2 | 14.29 | 375 | 46.2 |
| 9.4 | 9.39 | 187.5 | 23.1 |
| 10.9 | 8.12 | 62.5 | 7.7 |
| 11.5 | 7.71 | 125 | 15.4 |
| 12.1 | 7.33 | 156.25 | 19.2 |
| 14.4 | 6.14 | 343.75 | 42.3 |
| 15.6 | 5.68 | 250 | 30.8 |
| 16.5 | 5.38 | 375 | 46.2 |
| 17.6 | 5.02 | 187.5 | 23.1 |
| 18.2 | 4.86 | 375 | 46.2 |
| 19.4 | 4.57 | 812.5 | 100.0 |
| 20.0 | 4.43 | 500 | 61.5 |
| 22.9 | 3.87 | 187.5 | 23.1 |
| 24.7 | 3.60 | 250 | 30.8 |
The values of intensity (I) and relative intensity (rel I) of the peaks being likely to vary by ±15 %

4. The composition according to any of claims 1 to 3, comprising at least 99 % patent blue sodium salt and less than 0.5 % mono-desethyl impurities, the percentage being measured by high performance liquid chromatography with detection at 230 nm.

5. The composition according to any of claims 1 to 4, wherein no impurity other than the mono-desethyl impurities is contained in an amount higher than 0.15 %, the percentage being measured by high performance liquid chromatography with detection at 230 nm.

6. The composition according to claim 5, wherein no impurity other than the mono-desethyl impurities is contained in an amount higher than 0.1 %, the percentage being measured by high performance liquid chromatography with detection at 230 nm.

7. The composition according to any of claims 1 to 6, comprising less than 200 ppm of metal contaminants, advantageously less than 100 ppm of metal contaminants, better still less than 50 ppm of metal contaminants, and further advantageously less than 20 ppm of metal contaminants.

8. The composition according to any of claims 1 to 7 that is free of NaCl.

9. The composition according to any of claims 1 to 8 for use thereof as medicinal product.

10. The composition according to any of claims 1 to 8 for use thereof as diagnosis product.

11. The composition according to any of claims 1 to 8 for use thereof as contrast agent for the detection of lymphatic vessels and arterial territories and/or as diagnosis agent for cancer.

12. The composition according to any of claims 1 to 8 for use thereof according to claim 11 for the detection of the sentinel lymph node before biopsy in patients suffering from operable breast cancer, or for intra-operative mapping of the sentinel lymph nodes in patients suffering from cancer of the colon.

13. A method for producing a medicinal product or diagnosis product comprising the manufacture of the composition according to any of claims 1 to 8, and the insertion of said composition in a pharmaceutically acceptable carrier.

14. A composition in tablet or capsule form comprising from 1 to 500 mg of a composition according to any of claims 1 to 8 in a pharmaceutically acceptable carrier.
